# EUROPEAN PATENT APPLICATION

(11) **EP 2 808 043 A1**
(43) Date of publication of application: **03.12.2014**
(21) Application number: 14170440.3
(22) Date of filing: 28.05.2014
(51) Int. Cl.: A61M 1/16, B65D 81/00

(54) **Method to manufacture a bag containing bicarbonate powder**

(30) Priority: 28.05.2013 IT BO20130267
(71) Applicant: Bellco S.r.l., Mirandola (IT)
(72) Inventor: Scala, Maurizio, 98026 Nizza di Sicilia (IT); Lupotti, Marco, 41030 Sorbara di Bomporto (IT)
(74) Representative: Boggio, Luigi

(57) **Abstract**

A method to manufacture bags containing bicarbonate powder, each bag having two pockets (2, 3) communicating by means of a lower U-shaped channel (5), the bottoms (6, 7) of the pockets and the outer side (20) of the lower channel (5) defining the bottom of the bag (1). The method comprises the steps of longitudinally folding a plastic film, welding the folded film so as to form a semifinished product (31) comprising a succession of open bags, with the relative pockets (2, 3) arranged transverse to the folding line (32), cutting the semifinished product (31) so as to separate the open bags, and filling the pockets (2, 3) of each open bag with bicarbonate powder. Forming the semifinished product (31) comprises the steps of performing, along the folding line (32), a succession of first welds shaped as the bottom of the bags and performing a succession of second welds (13), which are transverse to the folding line (32), so as to divide the pockets (2, 3) of each pair of pockets (2, 3) from one another and to define the inner side (14) of the lower channel (5).

## Description

The present invention relates to a method to manufacture a bag containing bicarbonate powder, in particular a bag containing sodium bicarbonate powder, which can be connected to a dialysis machine so as to produce a saturated bicarbonate solution.

Containers are known that hold sodium bicarbonate powder and can be connected to the hydraulic circuit of the dialysis machine supplying a sodium bicarbonate solution to the dialysis filter so as to receive, from the hydraulic circuit, a solvent, typically water, and to supply, to the hydraulic circuit, a saturated bicarbonate solution produced by the dissolution of the bicarbonate powder in the solvent flowing through the container. These containers are disposable medical devices that, after having been used, are classified as special waste, namely waste that requires a specific disposing procedure, whose costs are substantially proportional to the volume of the material to be disposed of.

A type of container is known that holds bicarbonate powder and is made of a flexible plastic material in order to reduce manufacturing and transport costs and in order to ensure a limited size after the use. This flexible container basically consists of a plastic film bag containing bicarbonate powder and closed in a tight manner by a fitting provided with an inlet for the solvent and an outlet for the saturated bicarbonate solution. The container comprises, furthermore, a dip tube consisting of a straw, which, with one end, is connected to the outlet of the fitting and, with the other end, is dipped to the bottom of the bag so as to draw out the saturated solution without sucking in air, and a filter, which is connected to the dipped end of the straw so as to prevent bicarbonate powder from entering the hydraulic circuit of the dialysis machine. The fitting is provided with an additional mouth having a section that is larger than said inlet and outlet, so as to allow the bag to be filled with bicarbonate powder after the container has been assembled. After the filling with bicarbonate powder has ended, the additional mouth is closed.

The flexible container described above, when it is empty, after having been used, always takes up a significant space, even if it has been flattened, due to the presence of the straw and of the filter and due to the large size of the additional mouth. Furthermore, the presence of the straw and of the filter and the need to close the additional mouth after the bag has been filled are a possible source of complications for the container manufacturing method, which keep the manufacturing costs high.

Finally, despite the presence of the dip tube, a certain quantity of gas always enters the hydraulic circuit of the dialysis machine, this gas basically consisting of the carbon dioxide produced by the chemical reaction between the solvent and the bicarbonate powder and of the air contained in the particles of the bicarbonate powder. This gas is strongly undesired because dialysis liquid cannot contain gas and because the presence of gas bubbles in the hydraulic circuit can negatively affect the dynamics control function performed by the servosystems of the dialysis machine.

The object of the present invention is to provide a method to manufacture a bag containing bicarbonate powder, said method being conceived to eliminate the aforementioned drawbacks and, at the same time, to be performed in a straightforward and low-cost manner.

The present invention provides a method to manufacture a bag containing bicarbonate powder according to the appended claims.

The present invention will now be described with reference to the accompanying drawings, which show a nonlimiting embodiment thereof, wherein:
- figure 1 shows, in a plan view, a bag containing bicarbonate powder manufactured with the manufacturing method according to the present invention;
- figure 2 shows, in a perspective view, the bag of figure 1;
- figure 3 shows the lower part of the bag of figure 1, when the lower part is folded;
- figure 4 shows the flow chart of the method to manufacture the bag according to the invention;
- figure 5 shows a semifinished product obtained at an intermediate stage of the manufacturing method of figure 4;
- figure 6 shows, in a perspective view, a coupling assembly of a dialysis machine for the coupling of the connection of the bag of figure 1 to a hydraulic circuit of the machine for the distribution of a solvent and of a bicarbonate solution produced with said solvent;
- figures from 7 to 10 show, in a perspective view and partially in section, the coupling assembly of figure 6 in different operating configurations; and
- figure 11 shown, in a schematic manner, the hydraulic circuit of the dialysis machine and part of the coupling assembly of figure 6, which connects the bag of figure 1 to the hydraulic circuit.

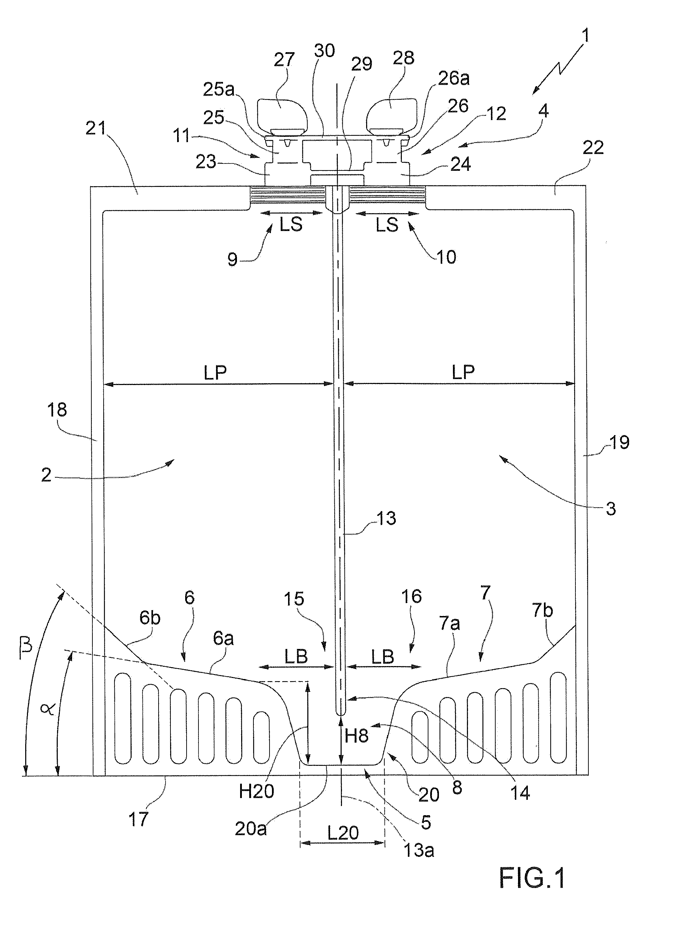

In figure 1, the bag manufactured with the method according to the present invention is indicated with number 1 and is shown empty and flattened on the plane of the plan view. The bag 1 is divided into two pockets 2 and 3, which are suited to contain bicarbonate powder, and comprises a lower U-shaped channel 5, which establishes a communication between the bottom portions, hereinafter simply referred to as bottoms 6 and 7, of the two pockets and has a central section 8 that is completely arranged under the minimum level of both bottoms 6 and 7, so that the lower channel 5 is normally full of bicarbonate powder, when, in use, the bag 1 is held with the bottoms 6 and 7 facing downwards. The pockets 2 and 3 and the lower channel 5 are manufactured as one single piece from a plastic film that is folded in two parts and welded. The plastic film is, for example, a polyamide-polyethylene (PA-PE) film. Each pocket 2, 3 is closed on the upper side, except for a respective upper opening 9, 10. The bag 1 comprises, furthermore, an upper connection 4 for the connection to the hydraulic circuit of a dialysis machine. The upper connection 4 comprises two passage elements 11 and 12, each of which is welded in a tight manner to the upper opening 9 and 10 of a respective pocket 2, 3, so as to allow a solvent, consisting for example of water, to be introduced into the bag (1) through one of the passage elements, for example element 11, and a saturated bicarbonate solution to flow out through the other passage element (12), after the solvent has spread through the bicarbonate powder from the pocket 2 to the pocket 3 flowing through the lower channel 5. The bag 1 lacks any kind of dip tube and relative filter.

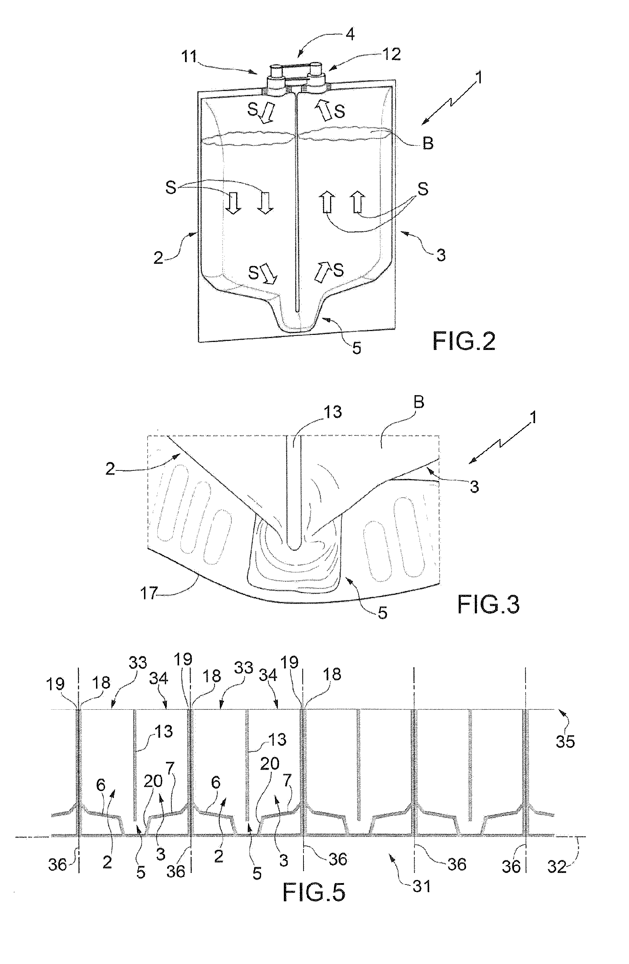

Figure 2 shows the bag 1 filled with sodium bicarbonate powder, indicated with B. The arrows indicated with S in figure 2 show the flow of the solvent, which flows in through the passage element 11, flows from the pocket 2 to the pocket 3, flows through the lower channel 5, and flows out of the passage element 12. The solvent consists, for example, of water. As the solvent flows in through the passage element 11, the saturated bicarbonate solution flows out of the other passage element 12.

With reference to figure 1, again, the lower channel 5 is delimited on the upper side by an inner side 14, which is convex, and on the lower side by an outer side 20, which is substantially concave. The two pockets 2 and 3 are symmetrical relative to a longitudinal symmetry axis 13a of the bag 1 and have the same capacity. In particular, the bag 1 has a central welded joint 13, which extends along the axis 13a so as to divide the bag 1 into the two pockets 2, 3 and so as to define, with an end of its, the inner side 14 of the lower channel 5. The bottoms 6 and 7 of the pockets 2 and 3 have, in symmetrical and close positions relative to the axis 13a, respective lower openings 15 and 16 that are contiguous with said central welded joint 13. The lower channel 5 is symmetrical relative to the axis 13a, as well, and establishes a communication between the lower openings 15 and 16. In other words, each one of the two branches of the lower channel 5 communicates with the lower opening 15, 16 of the respective pocket 2, 3.

The bottoms 6 and 7 are inclined towards the respective lower openings 15 and 16 in a specular manner relative to the axis 13a. In particular, each bottom 6, 7 comprises a first straight portion 6a, 7a, which is joined to the respective lower opening 15, 16 and is inclined, relative to a direction defined by the straight lower edge 17 of the bag 1, with an angle α ranging from 5° to 15°, and a second portion 6b, 7b, which extends from a respective lateral welded joint 18, 19 of the bag 1, which defines the outer side of the respective pocket 2, 3, to the first portion 6a, 7a and is inclined, relative to the lower edge 17, with an angle β ranging from 40° to 50°. As one can assume from figure 1, the lateral welded joints 18 ans 19 are parallel to the welded joint 13 and the lower edge 17 is perpendicular to the axis 13a.

Each lower opening 15, 16 having a width LB, which is measured perpendicular to the axis 13a and in the flattening plane of the bag 1 and is smaller than the half, and in particular smaller than one third, of the inner width LP of a pocket 2, 3, which is measured perpendicular to the axis 13a, as well. The first portion 6a, 7a of each bottom 6, 7 is joined to an end segment of the outer side 20 of the channel 5. The outer side 20 is substantially concave, in that it comprises a straight central segment defining the bottom 20a of the channel. The bottom 20a has a width L20, which is measured perpendicular to the axis 13a and is larger than the width LB, and in particular equal to approximately one third of the width LP. Each one of the two branches of the channel 5 has a length H20, which is measured parallel to the axis 13a between the lowest point of the straight portions 6a and 7a of the bottoms 6 and 7 and the bottom 20a of the channel 5 and is approximately equal to the width L20, in particular equal to approximately one third of the width LP. The central section 8 of the channel has a depth H8, which is measured along the axis 13a and is smaller that two thirds of the length H20.

The bottoms 6 and 7 and the outer side 20 of the channel 5 define a bottom line of the bag 1, which is obtained by welding, according to a corresponding shape, the lower part of the bag 1, namely the part that originates from the folding of the plastic film along the edge 17.

The upper openings 9 and 10 are in symmetrical and close positions relative to the axis 13a and are contiguous with the central welded joint 13. Each pocket 2, 3 comprises two upper flaps, which are partly welded to one another so as to form a respective upper welded joint 21, 22, that is contiguous with a respective lateral welded joint 18, 19, and, for the remaining part, define the respective opening 9, 10. Each upper opening 9, 10 has a width LS, which is measured perpendicular to the axis 13a and is substantially equal to approximately one third of the length LP.

The central welded joint 13 has a width, which is measured perpendicular to the axis 13a and ranges from 3 to 7 mm. The lateral welded joints 18 and 19 have a width that slightly larger than the one of the central welded joint 13, for example ranging from 4 to 8 mm. The upper welded joints 21 and 22 have a width, which is measured parallel to the axis 13a and ranges from 10 to 14 mm.

The passage elements 11 and 12 have a substantially circular symmetry and are identical to one another. Each passage element 11, 12 comprises a respective base portion 23, 24, whose outer peripheral surface is tightly welded to the edges of the respective upper opening 9, 10, and a respective end portion 25, 26, which is closed, on the upper side, by a respective plug 27, 28 that can be removed by hand, by breaking it, so as to permit the connection to a respective branch of a hydraulic circuit (not shown) of a dialysis machine.

The connection 4 comprises, furthermore, two flat elements 29 and 30, which are parallel to one another and substantially perpendicular to the axis 13a, so as to rigidly connect the passage elements 11 and 12 to one another, keeping a given distance. In particular, the flat element 29 connects the base portions 23 and 24, while the other flat element connects the end portions 25 and 26. The end portions 25 and 26 have respective annular edges 25a and 26a, which are coplanar and joined to the flat element 30. The connection 4 is entirely made of an injection-moulded plastic material.

In the example of figure 1, the dimensional parameters of the bag 1 are the following:
- overall height of the bag, measured along the axis 13a, excluding the connection 4, equal to 295 mm;
- overall width of the bag, measured transversely to the axis 13a, equal to 250 mm;
- width LP approximately equal to 116 mm;
- width L20 equal to 42 mm;
- height H20 equal to 42 mm;
- depth H8 equal to 25 mm;
- width LS equal to 38 mm;
- angle α approximately equal to 10°;
- angle β approximately equal to 45°;

The bag 1 of the example of figure 1 is shaped so as to hold 1300 g of sodium bicarbonate powder. For smaller quantities of bicarbonate, the bag 1 is similar to the one of figure 1, with the only difference that it has a smaller overall height.

The shape of the bag 1 is divided into the two pockets 2 and 3, which communicate at the bottom by means of the channel 5, which acts as a communicating vessel, thus eliminating the need for a dip tube and a relative filter. The absence of dip tubes and filters allows manufacturers to produce a bag that, after having been used and flattened, takes up a very small place. Furthermore, thanks to the lack of a dip tube, the number of components making up the bag 1 is significantly reduced: plastic film, bicarbonate powder, and the connection 4 for the coupling to the dialysis machine.

Thanks to the particular configuration described above, the channel 5 never completely closes during the use of the bag 1, since, when the bag 1 filled with bicarbonate powder B folds in correspondence to the channel 5, different folding lines are formed therein, which always leave a free passage between the two pockets 2 and 3, as one can assume from the example of figure 3. Furthermore, the relatively small sizes of the channel 5 allow the residual quantity of bicarbonate left in the bag at the end of a dialysis treatment to be minimized. As a matter of fact, the residual quantity of bicarbonate is substantially the one located in the trap defined by the portion of the channel 5 arranged under the imaginary line defined by the depth H8.

The absence of a dip tube permits another advantage, which is that of further simplifying the process performed to assemble and fill the bag 1.

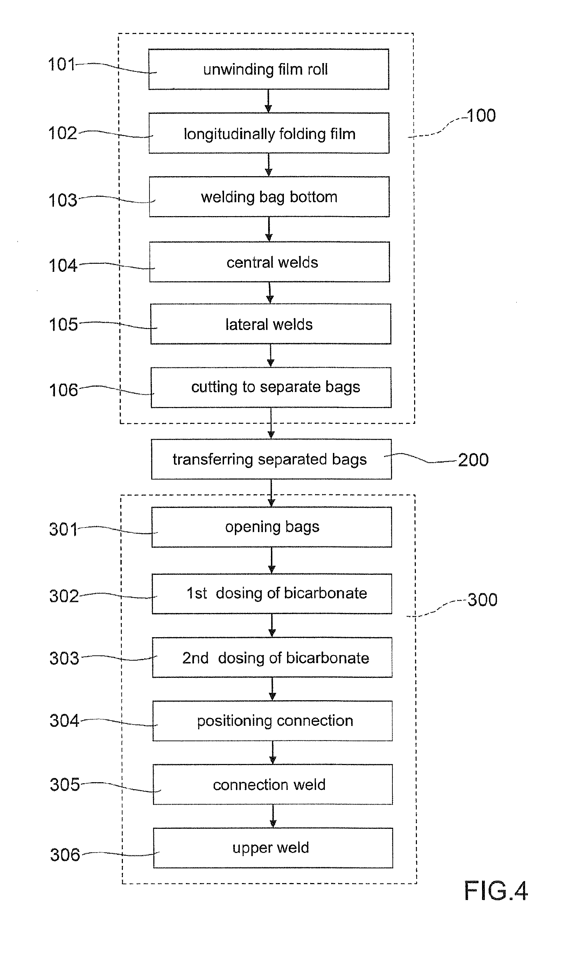

According to the present invention, the method used to manufacture the bag 1 is implemented by an automatic apparatus of the FFS type (Form, Fill and Seal) comprising a series of operating stations controlled by a control unit. Figure 4 shows the sequence of the main processing steps performed by the manufacturing apparatus. Each one of the steps shown can be carried out by a respective operating station of the manufacturing apparatus. Figure 5 shows a semifinished product at an intermediate stage of the method to manufacture the bag 1.

With reference to figures 4 and 5, the production of the bag starts by unwinding the plastic film from a roll (step 101). The plastic film is folded along its longitudinal middle line while it is fed (step 102). The folded plastic film is fed discontinuously by means of a system of friction rollers. The feeding pitch of the friction roller system is equal to the overall width of a bag 1.

At this point, the folded film is welded in different parts so as to form a semifinished product 31 (figure 5), which comprises a succession of open bags, each of which consists of a respective pair of pockets (2, 3) and of the relative channel 5 of the bag 1, the pockets 2 and 3 being arranged transverse to the folding line 32 and being partially welded so as to be open in correspondence to respective upper sides 33 and 34, which are aligned along a side 35 of the folded film that is opposite to the side defined by the folding line 32.

In particular, on the side of the folding line 32, a series of welds having a predefined profile are performed in order to form the bottom of the bags (step 103), namely to form the bottom line of each bag 1 defined by the bottoms 6 and 7 of the pockets 2 and 3 and by the outer side 20 of the channel 5. Then, one needs to perform a series of straight welds transverse to the folding line 32, which are alternated, in the direction of the folding line 32, with the welds 13 and are parallel thereto and each correspond to the central weld 13 of a respective bag 1 (step 104), and a further series of straight welds transverse to the folding line 32, each of which correspond to the joining of the lateral welds 18 and 19 of two bags 1 that are contiguous with one another (105).

Each weld 13, each pair of welds 18 and 19 as well as each shaped weld of the bottom of the bag 1 are performed one at a time in correspondence to relative welding stations. Each welding step is performed by means of a respective group of plates heated by electrical resistors and having the shape of the welds to be performed. Furthermore, each welding step is followed by a respective weld cooling step.

Finally, the semifinished product 31 is cut along cutting lines 36 (figure 5) coinciding with the middle lines of the transverse welds 18, 19, so as to separate the open bags from one another (step) 106, namely so as to separate the pairs of pockets 2, 3 from one another in such a way that they are laterally defined by the respective lateral welded joints 18 and 19.

Steps 101-106, which are indicated as a whole with number 100 in figure 4, are carried out in a series of stations arranged in a line and run through by a linear conveying system which feeds, at first, the folded plastic film, then the semifinished product and in the end the separated open bags. The following processing steps, which are indicated as a whole with number 300, are carried out in a series of stations arranged around a carousel conveyor and substantially deal with the filling of the pockets 2 and 3 with the bicarbonate powder and with the closing thereof with the insertion of the relative connection 4 for the connection to the dialysis machine. The separated open bags are transferred, on by one, from the linear conveying system to the carousel conveyor by means of a mechanical hand (step 200).

In particular, after having been placed on the carousel conveyor, the pockets 2 and 3 are opened starting from the upper sides 33 and 34, namely the flaps of the upper side 33, 34 of each pocket 2, 3 are mutually moved apart so as to allow the open bag to be filled (step 301), and then they are simultaneously filled with bicarbonate powder, thus introducing the latter through the upper sides 33 and 34 by means of two subsequent dosing steps (steps 302 and 303). For each open bag that has been filled, the connection 4 is positioned with the respective passage elements 11 and 12 in the two upper sides 33 and 34 (step 304), the connection 4 is welded to part of the flaps of the upper sides 33 and 34 (step 305) and the remaining part of the flaps of the upper sides 33 and 34 is welded so as to completely close the pockets (step 306), namely by forming the welded joints 21 and 22 shown in figure 1, thus obtaining the finished bag 1.

The bag 1 obtained with the manufacturing method described above can be used with a dialysis machine, which, in the figures from 6 to 11, is indicated as a whole with number 37.

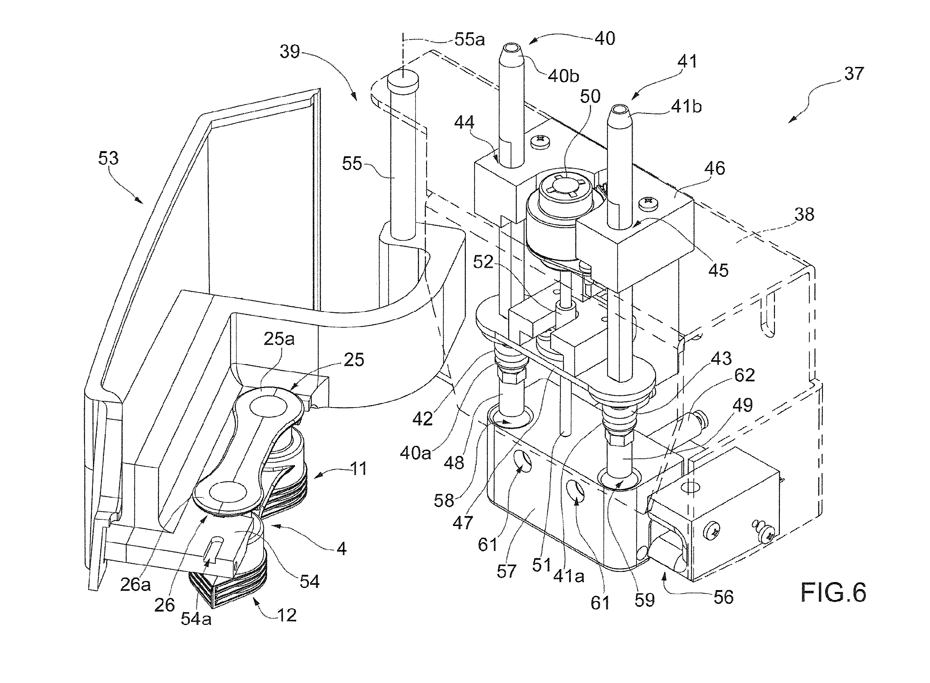

With reference to figure 6, the dialysis machine 37 comprises a frame 38 and a coupling assembly 39, which is mounted on the frame 38 so as to receive the connection 4 of the bag 1 and couple the connection 4 to a hydraulic circuit (not shown) of the machine 37, said circuit being suited to feed a solvent to the bag 1 and to draw, from the bag 1, a sodium bicarbonate solution obtained from the dissolution, inside the bag 1, of the bicarbonate powder in the solvent. For the sake of simplicity, figure 6 only shows the connection 4 of the bag 1.

The coupling assembly 39 comprises two ducts 40 and 41, each of which comprises a respective coupling end 40a, 41a, which is provided with at least one sealing ring 42, 43 so as to engage, in a tight and removable manner, the end portion 25, 26 of a respective passage element 11, 12 of the connection 4. The other ends 40b and 41b of the ducts are permanently connected to the hydraulic circuit. The ducts 40 and 41 can slide in two respective vertical circular guides 44 and 45, obtained in a sub-frame 46 fixed to the frame 38, so as to be able to vertically move between a raised waiting position, which is the one shown on figure 6, and a lowered operating position. The coupling ends 40a and 41a are rigidly joined by means of a horizontal bracket 47, so that the distance between the ducts 40 and 41 is equal to the distance between the passage elements 11 and 12 of the bag 1.

The two ducts 40 and 41 comprise two respective filters 48 and 49 so that, during the coupling to the respective passage elements 11 and 12, bicarbonate powder available inside the bag 1 is prevented from entering the hydraulic circuit. In particular, each filter 48, 49 is partially fitted into the coupling end 40a, 41a of the respective duct 40, 41 in such a way that a prevailing portion of the filter 48 and 49 projects from the coupling end 40a, 41a so as to be able to penetrate the respective passage element 11, 12, when the latter is engaged by the coupling end 40a, 41a of the relative duct 40, 41.

The coupling assembly 39 comprises a motor 50, which is mounted on the sub-frame 46 and whose drive shaft ends with a worm screw 51, and a threaded element 52, which is fixed to the bracket 47 and coupled to the worm screw 51 so as to allow the ducts 40, 41 to move between the raised position and the lowered position.

The coupling assembly 39 comprises, furthermore, a cover 53, which is hinged to the frame 38 and is provided with a support 54, which is suited to receive the connection 4 of the bag 1. The hinge 55 connecting the cover 53 to the frame 38 has a substantially vertical axis 55a. The support 54 consists of a U-shaped plate, which remains horizontal during the rotation of the cover 53 relative to the axis 55a and is suited to receive the end portions 25 and 26 of the passage elements 11 and 12 of the connection 4, so that the annular edges 25a and 26a rest against the edge of the plate. The cover 53 can be manually moved between an open position, which is the one shown in figure 6 and in which the bag 1 can be loaded by inserting the connection 4 into the support 54, and a closed position, in which the passage elements 11 and 12 are in position, coaxial with the respective ducts 40 and 41, and the panel of the cover 53 covers the ducts 40 and 41 on the outside. The coupling assembly 39 comprises a hooking means 56, which is suited to be coupled, in a releasable manner, to a recess 54a obtained in the support 54, so as to hold the cover 53 in the closed position.

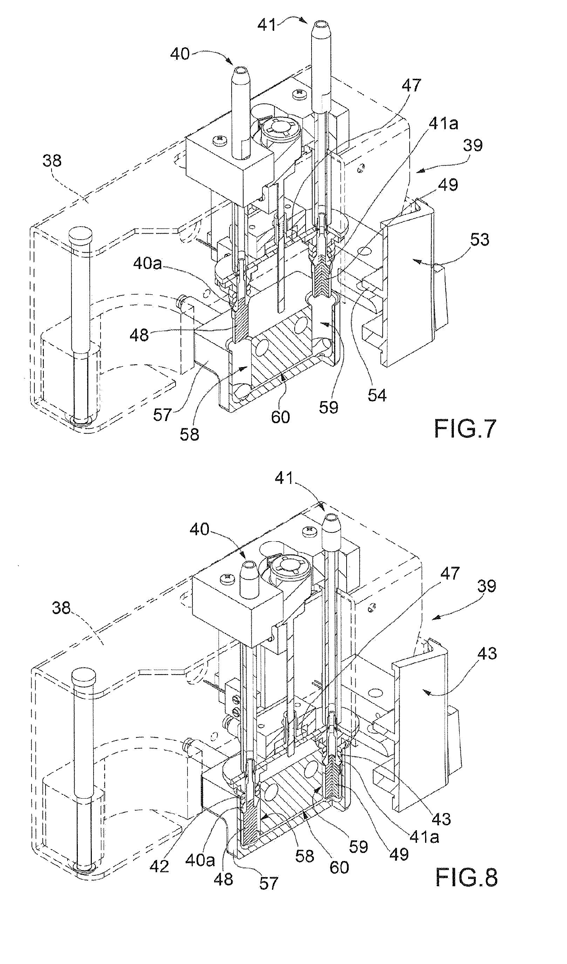

The dialysis machine 37 comprises a solid body 57, which has two vertical channels 58 and 59, having a distance that is equal to the one of the ducts 40 and 41, and has an inner duct 60 (figures 7 and 8), which establishes a mutual communication between the bottoms of the two channels 58 and 59. The body 57 has two holes 61, which are suited to be engaged, in a sliding manner, by two respective horizontal pins 62 fixed to the frame 38, so as to allow the body to horizontally move between an operating position, which is the one shown in figure 6 and in which each channel 58, 59 is coaxial to a respective duct 40, 41, and a retracted position, in which the body 57 leaves room for the connection 4. The body 57 is normally held in the operating position by an elastic element (not shown). The channels 58, 59 and 60 define a by-pass path, which is suited to close the hydraulic circuit of the dialysis machine 37, so as to allow the circuit itself to be washed.

Figure 7 shows, in a perspective view partially in section along the longitudinal axes of the ducts 40 and 41, the coupling assembly 39 in a by-pass path loading configuration, in which the channels 40 and 41 are in the raised waiting position, the body 57 is in the operating position, the bag is not inserted into the support 54, and the cover 53 is in the closed position. The U shape of the support 54, which is not completely visible in figure 7, has a width that is such as to embrace, with precision but without interference, the body 57, when the cover 53 is in the closed position.

Figure 8 shows, in the same view as figure 7, the coupling assembly 39 in a by-pass path sealing configuration, which differs from the configuration of figure 7 due to the fact that the ducts 40 and 41 are in the lowered operating position, with the coupling ends 40a and 41a engaging the respective channels 58 and 59, so that the filters 48 and 49 penetrate the channels 58 and 59 up to the level of the channel 60. The sealing rings 42 and 43 ensure a tight sealing between the outer surface of the coupling ends 40a and 41a and the inner surface of the channels 58 and 59. The washing of the hydraulic circuit is performed when the coupling assembly 39 is in the by-pass path sealing configuration.

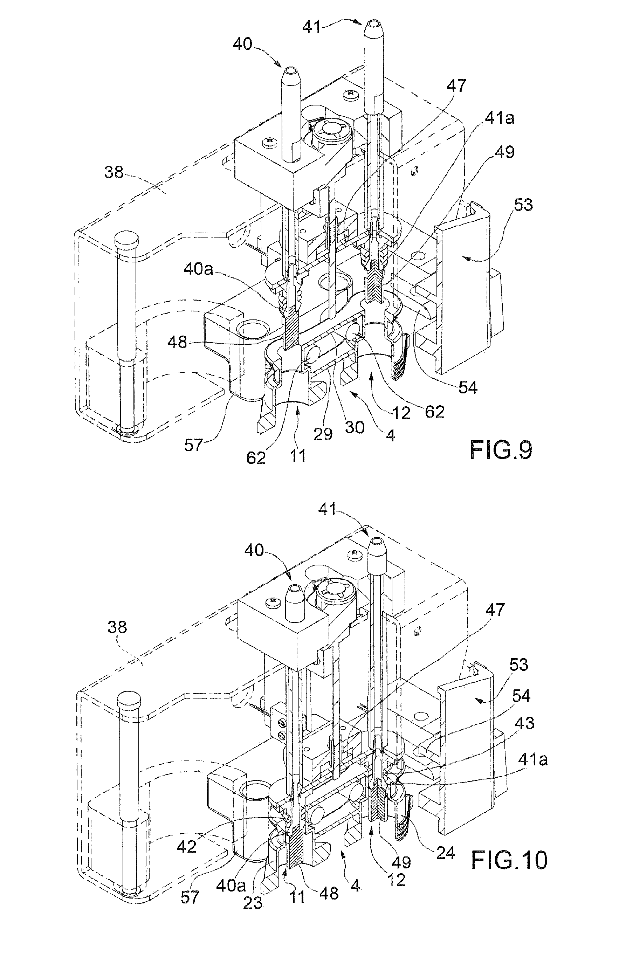

Figure 9 shows, in the same view as figure 7, the coupling assembly 39 in a bag 1 loading configuration, which differs from the configuration of figure 7 due to the fact that the connection 4 of the bag 1 is inserted into the support 54 and the cover 53, in the closed position, presses the connection 4 against the body 57 so as to hold the latter in the retracted position. The body 57, in the retracted position, leaves the pins 62 uncovered, which place themselves between the flat elements 29 and 30.

Figure 10 shows, in the same view as figure 7, the coupling assembly 39 in a bag 1 sealing configuration, which differs from the configuration of figure 9 due to the fact that the ducts 40 and 41 are in the lowered operating position, with the coupling ends 40a and 41a engaging the respective passage elements 11 and 12 of the connection 4, so that the filters 48 and 49 penetrate until they reach the inside of the base portions 23 and 24 of the passage elements 11 and 12. The sealing rings 42 and 43 ensure a tight sealing. When the coupling assembly 39 is in the bag 1 sealing configuration, the hydraulic circuit feeds the solvent to the bag 1 through, for example, the duct 40, which is tightly connected to the passage element 11, and the sodium bicarbonate solution is drawn from the bag 1 through the duct 41, which is tightly connected to the passage element 12.

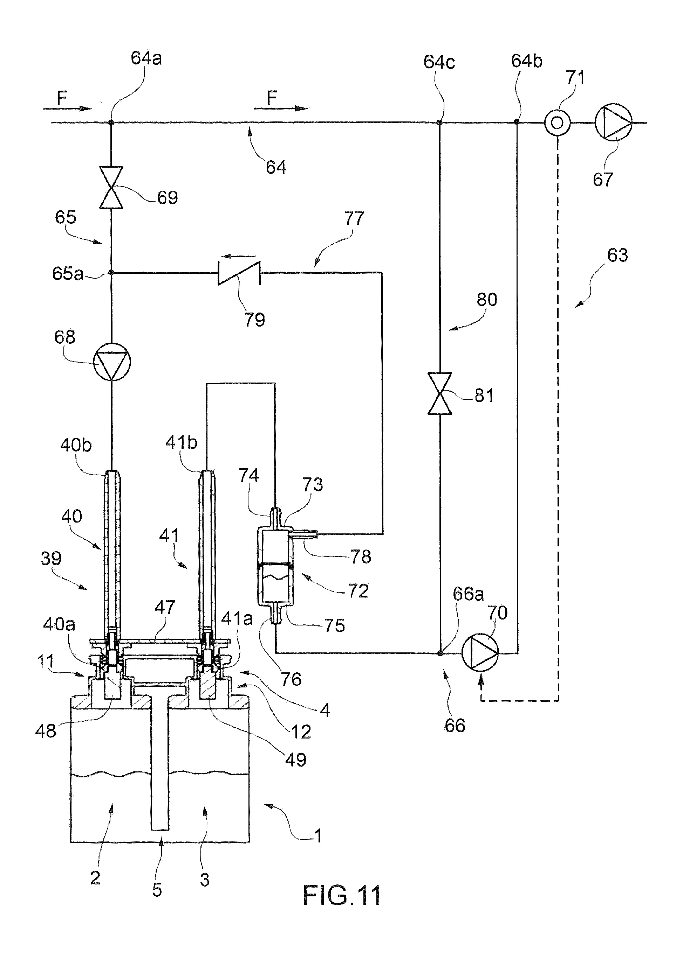

In figure 11, number 63 indicates, as a whole, the hydraulic circuit of the dialysis machine 37 for the distribution of a solvent and of a bicarbonate solution produced with said solvent. In particular, the circuit 63 is suited to feed a solvent to the bag 1 and to draw, from the bag 1, a saturated sodium bicarbonate solution produced, inside the bag 1, by the dissolution of the bicarbonate powder in the solvent. The circuit 63 comprises a main branch 64, which is flown through by the solvent according to a given circulation direction F, a solvent delivery branch 65, which connects a point 64a of the main branch 64 to the end 40b of the duct 40 of the coupling assembly 39 (figure 6), so as to the feed the solvent to the bag 1, and a solution return branch 66, which connects the end 41b of the duct 41 of the coupling assembly 39 to another point 64b of the main branch 64 arranged downstream of the point 64a relative to the circulation direction F, so as to receive the solution from the bag 1 and introduce the solution into the main branch 64. The outlet of the main branch 64, arranged downstream of the point 64b relative to the circulation direction F, supplies the bicarbonate solution to the dialysis filter (not shown).

The main body 64 comprises a pump 67, which is arranged downstream of the point 64b relative to the circulation direction F, so as to create a head that is such as to cause the solvent to circulate according to the direction F, oriented from the point 64a to the point 64 b.

The solvent delivery branch 65 comprises another pump 68 to push the solvent into the duct 40 and, thus, into the bag 1. Furthermore, the solvent delivery branch 65 comprises a pressure relief valve 69, which is arranged upstream of the pump 68 so as to limit the pressure of the solvent in the bag 1. The pressure in the bag 1 must be lower that 0.3 bar (30 kPa), otherwise there is a risk of explosion.

The solution return branch 66 comprises a pump 70 to push the solution towards the main branch 64 during the steady-state operation of the dialysis machine 37. The bicarbonate solution that reaches the solution return branch 66 mixes with the solvent coming from point 4a in the point 64b of the main branch 64. Therefore, the bicarbonate percentage dissolved in the solution at the outlet of the main branch 64, namely downstream of the point 64b relative to the circulation direction F, is linked to the bicarbonate percentage of the solution in the solution return branch 66.

The main branch 64 comprises, downstream of the point 64b relative to the circulation direction F, and in particular between the point 64b and the inlet of the pump 67, conductivity sensor 71 to provide a signal indicating the conductivity of the solution at the outlet of the main branch 64. The conductivity of the solution is liked to the percentage of bicarbonate dissolved. The pump 70 is controlled as a function of the signal provided by the conductivity sensor 71, so as to keep the percentage of bicarbonate downstream of the point 64b at a desired value.

The solvent return branch 66 comprises a dripper 72 to prevent the gases generated with the production of the solution to be introduced into the main branch 64. The gases generated during the production of the solution comprise carbon dioxide, which is a product of the reaction between the solvent and the bicarbonate powder, and air in the form of bubbles, which is released by the breaking of the particles of the bicarbonate powder when the latter is dissolved in the solvent.

The dripper 72 is arranged between the duct 41 and the pump 70. In particular, the dripper 72 consists of a container having a preferably - but not necessarily-cylindrical shape, whose ceiling 73 has an inlet 74 that is connected to the end 41b of the duct 41 and whose bottom 75 has an outlet 76 that is connected to the inlet of the pump 70. The dimensions of the dripper 72 are chosen as a function of the capacity of the bag 1. During the steady-state operation, the dripper 72 must remain substantially full of solution, so as to compensate possible emptying events caused by the air bubbles coming from the bag 1. Advantageously, the dripper 72 has a capacity ranging from 5 to 20 cm³. Higher capacity values are not recommended, because they would excessively increase the washing times of the circuit 63.

The circuit 63 comprises a gas recirculation branch 77, which connects a further outlet 78 of the dripper 72, obtained in the ceiling 73 of the dripper 72, and a point 65a of the solvent delivery branch 65, arranged between the pump 68 and the pressure relief valve 69, so as to introduce the gases generated with the production of the solution into the solvent delivery branch 65. The gas recirculation branch 77 comprises a non-return valve 79 to prevent the solvent circulating in the solvent delivery branch 65 to directly flow into the solution return branch 66 without flowing through the bag 1. The gas recirculation branch 77 brings the gases gathered in the dripper 72 back into the bag 1, so as to prevent the gases themselves from flowing through the pumps 70 and 67 and entering the main branch 64, whose outlet is connected to the dialysis filter.

The circuit 63 comprises, furthermore, a further solution return branch 80, which connects a point 66a of the other solution return branch 66 to a point 64c of the main branch 64 arranged upstream of the point 64b and comprises an on/off valve 81, which is controlled by the control unit (not shown) of the dialysis machine 37 so as to define a by-pass path for the solution that allows the initial filling and the final emptying of the bag 1 to be quickened. In particular, the point 66a is arranged between the outlet 76 of the dripper 77 and the inlet of the pump 70 and the point 64c is arranged between the point 64a and the point 64b.

In use, the on/off valve 81 is opened for an initial period so as to allow both pockets 2 and 3 of the bag 1 to be quickly filled with the solvent. A the end of the initial period, namely when the pockets 2 and 3 are presumably full of solvent, a steady-state operating period starts, during which the on/off valve 81 is closed so as to cause the solution to circulate only in the solution return branch 66, so that the flow solution directed at the main branch 64 is regulated by the pump 70. A the end of the steady-state operating period, namely when the bicarbonate of the bag 1 is close to being completely used up, a final period starts, during which the on/off valve 81 is reopened so as to allow the bag 1 to be quickly emptied.

According to a further embodiment of the circuit 63, which is not shown, the pump 67 is arranged upstream of the conductivity sensor 71, in particular between the point 64b and the conductivity sensor 71.

According to a further embodiment of the circuit 63, which is not shown, the solvent delivery branch 65 comprises an adjusted narrowing, which is arranged between the point 65a and the inlet of the pump 68, so as create a vacuum upstream of the pump 68 that is such as to draw gas or gas mixed with solution from the dripper 72.

One of the advantages of the dialysis machine 37 is that it permits the use of sodium bicarbonate bags without filters, such as bag 1, since the dialysis machine 37 already mounts the filters 48 and 49 for the bicarbonate powder on board the coupling assembly 39. Another advantage is that the gases generated with the production of sodium bicarbonate are prevented from flowing into the main branch 64 of the circuit 63, thanks to the presence of the dripper 72 on the solution return branch 66. Another advantage is a higher speed in the bag 1 filling and emptying steps, thanks to the presence of the further solvent return branch 80 provided with the on/off valve 81.

## Claims

1. A method to manufacture a bag containing bicarbonate powder, the bag (1) lacking any kind of dip tube and/or filter and comprising two pockets (2, 3), which are arranged one next to the other, have respective bottom portions (6, 7) and contain bicarbonate powder (B), and a lower U-shaped channel (5), which establishes a communication between said bottom portions (6, 7) and comprises an inner side (14) and a lower side (20), so that the bag (1) has a bottom defined by said bottom portions (6, 7) and by said outer side (20); the method comprising:
- folding (102) a plastic film along its own longitudinal middle line (32);
- welding (103-105) the folded film so as to form a semifinished product (31) comprising a succession of open bags, each consisting of a respective pair of pockets (2, 3) and of the relative lower channel (5), the pockets (2, 3) being arranged transverse to the folding line (32) and having respective upper open sides (33, 34);
- cutting (106) said semifinished product (31) so as to separate the open bags from one another;
- filling (301-303) the pockets (2, 3) of each open bag with bicarbonate powder through said upper open sides (33, 34); and
- closing (304-306) each filled bag on the upper side by means of welding;
welding (103-105) the folded film so as to form a semifinished product (31) comprising:
- performing (103), along the folding line (32), a succession of first welds having a predefined profile, so as to form the bottom of the bags; and
- performing (104) a succession of second welds (13), which are transverse to the folding line (32), so as to divide the pockets (2, 3) of each pair of pockets from one another and to define said inner side (14).

2. A method according to claim 1, wherein said second welds (13) are performed after said first welds.

3. A method according to claim 1 or 2, wherein welding (103-105) the folded film so as to form a semifinished product (31) comprises:
- performing (105) a succession of third welds (18, 19), which completely extend through the folded film transverse to said folding line (32) so as to define said open bags;
said semifinished product (31) being cut along said third welds (18, 19) so as to separate the open bags from one another.

4. A method according to claim 3, wherein said third welds (18, 19) are performed after said second welds (13).

5. A method according to claim 3 or 4, wherein said third welds (18, 19) are alternated with said second welds (13) in the direction of said folding line (32).

6. A method according to any of the claims from 1 to 5, wherein said bag (1) comprises an upper connection (4), which is suited to connect the bag (1) to a dialysis machine (37) and is provided with two passage elements (11, 12) communicating, each, with a respective one of said pockets (2, 3); closing (304-306) each filled bag on the upper side by means of welding comprising:
- for each open bag that has been filled, positioning (304) said upper connection (4) with the respective passage elements (11, 12) on the two upper open sides (33, 34) of the pockets (2, 3); and
- welding (305) the passage elements (11, 12) to a part of the upper open sides (33, 34) of the pockets (2, 3) and welding (306) the remaining part of the upper open sides (33, 34) so as to completely close the pockets (2, 3).

7. A method according to any of the claims from 1 to 6, wherein each one of the welding steps (103-105, 305, 306) is performed by means of a respective group of plates heated by electrical resistors.
